# EUROPÄISCHE PATENTSCHRIFT

(11) **EP 3 570 957 B1**
(45) Veröffentlichungstag und Bekanntmachung des Hinweises auf die Patenterteilung: **13.01.2021**
(21) Anmeldenummer: 18701442.8
(22) Anmeldetag: 22.01.2018
(51) Int. Cl.: B01D 39/16, C12H 1/07, C12N 1/06

(54) **FILTERHILFSMITTELFREIES VERFAHREN ZUR ABSCHEIDUNG VON HEFE AUS HEFEHALTIGEN FLÜSSIGKEITEN, INSBESONDERE GETRÄNKEN**
FILTERING-AID-FREE METHOD FOR SEPARATING YEAST OUT OF YEAST-CONTAINING LIQUIDS, IN PARTICULAR BEVERAGES
PROCÉDÉ EXEMPT D'AUXILIAIRE DE FILTRATION POUR SÉPARER DE LA LEVURE DE LIQUIDES CONTENANT DE LA LEVURE, EN PARTICULIER DES BOISSONS

(30) Priorität: 20.01.2017 DE 102017000534
(43) Veröffentlichungstag der Anmeldung: 27.11.2019
(73) Patentinhaber: Schulze, Thomas, 94447 Plattling (DE)
(72) Erfinder: Schulze, Thomas, 94447 Plattling (DE)
(74) Vertreter: Beyer, Andreas
(86) Internationale Anmeldenummer: PCT/EP2018/051420
(87) Internationale Veröffentlichungsnummer: WO 2018/134396

(56) Entgegenhaltungen:
- WO-A1-98/45029
- WO-A1-03/095078
- DE-A1- 4 125 594
- DE-A1- 4 311 297
- DE-A1- 10 108 957
- JP-A- S63 278 517

## Beschreibung

Die vorliegende Erfindung betrifft ein Verfahren zur Hefeabscheidung aus hefehaltigen Flüssigkeiten, insbesondere Getränken, sowie die Verwendung eines sogenannten Tiefenfiltermediums zum Abscheiden von Hefe aus Getränken.

Bei der Herstellung alkoholischer Getränke wie z. B. Bier und Wein wird Hefe zugesetzt, damit ein als alkoholische Gärung bezeichneter biochemischer Prozess ablaufen kann, durch den Zucker zu Alkohol und Kohlendioxid umgesetzt wird. Nach dem Stoppen der alkoholischen Gärung wird die im Sud befindliche Hefe in der Regel abgetrennt, jedoch kann sie bei bestimmten alkoholischen Getränken wie beispielsweise Hefeweißbier auch im Getränk belassen werden.

Die nach Abschluss der alkoholischen Gärung erhaltene, hefehaltige Flüssigkeit, die üblicherweise zur Nachreifung für einige Wochen in einem Gärtank oder Lagertank verbleibt, wird als Unfiltrat bezeichnet. Zur Abtrennung der Hefe aus dem Unfiltrat stehen heutzutage verschiedene Systeme zur Verfügung. Eine Gruppe von Systemen basiert auf dem Prinzip der sogenannten Anschwemmfiltration, d. h. auf dem verwendeten Filtermedium muss sich zunächst ein Filterkuchen mit einer bestimmten Dicke bilden, bevor der Filtrationsvorgang seine volle Wirkung erreicht. Der eigentliche Filtrationsvorgang findet somit im Filterkuchen statt, während das Filtermedium lediglich als Stützsubstrat für den Filterkuchen dient. Während des Filtrationsvorgangs erhöht sich die Filterkuchendicke fortlaufend. Damit der Filterkuchen mit zunehmender Filterkuchendicke dem Unfiltrat keinen zu hohen Wiederstand entgegensetzt, werden dem Unfiltrat Filterhilfsmittel wie Kieselgur, Zellulose, Perlite etc. zugesetzt, die sich in den Filterkuchen einlagern und verhindern, dass das Filtersystem sich zusetzt.

Filtersysteme, die nach dem Prinzip der Anschwemmfiltration mit Filterhilfsmitteln arbeiten, können Kantenspaltfilter in Kerzenform, horizontale Zentrifugalfilter mit Metallgewebe und Rahmenfilter mit einer Anschwemmstützschicht sein, um nur einige Beispiele zu nennen. All diesen Anschwemmfiltrationssystemen ist gemeinsam, dass der Filterkuchen nach einer bestimmten Filtrationsdauer entfernt werden muss, was entweder durch Öffnen des Filtersystems und Ausspülen desselben geschehen kann, wie etwa bei den in der Getränkeindustrie häufig verwendeten Rahmenfiltern, oder durch Rückspülen des Filtersystems, d. h. durch Spülen des Filtersystems entgegengesetzt zur Filtrationsrichtung. Die Investitionskosten für Filtersysteme, die nach dem Prinzip der Anschwemmfiltration arbeiten, sind erheblich und betragen beispielsweise für einen in einer Bierbrauerei üblichen Rahmenfilter 150.000,- bis 200.000,- €.

Eine weitere Gruppe von Filtersystemen zur Abscheidung von Hefe aus hefehaltigen Flüssigkeiten kommt ohne Filterhilfsmittel aus. Zu dieser Gruppe gehören beispielsweise Membranfilter, insbesondere sogenannte Crossflow-Filter mit einer PES-Membran, aber auch Modulfilter aus Kieselgurschichten. Letztere sind nicht regenerierbar, denn die abgeschiedene Hefe setzt sich in den Kieselgurschichten fest und könnte nur durch Spülen mit einer Lauge wieder entfernt werden, was jedoch zur Zerstörung des Kieselgurmaterials führen würde und daher ausscheidet. Membranfilter setzen sich nach einer bestimmten Betriebsdauer zu und müssen ebenfalls aufwendig gereinigt werden. Crossflow-Membranfilter sind darüber hinaus regelungstechnisch aufwendig und somit teuer.

Die DE10108957A1 offenbart ein Verfahren zum Filtrieren von Flüssigkeiten, insbesondere Getränken, mit einer Filtereinrichtung, die insbesondere Tiefenfilterkerzen umfasst.

Der Erfindung liegt die Aufgabe zugrunde, ein Verfahren zur Hefeabscheidung aus hefehaltigen Flüssigkeiten anzugeben, das im Vergleich zu herkömmlichen Filtersystemen deutlich niedrigere Investitionskosten erfordert, das eine zumindest ebenso gute Filterwirksamkeit wie die herkömmlichen Filtersysteme gewährleistet und das eine einfache und kostengünstige Regeneration des Filtermediums ermöglicht.

Zur Lösung der vorgenannten Aufgabe schlägt die Erfindung ein Verfahren zur Hefeabscheidung aus hefehaltigen Flüssigkeiten, insbesondere Getränken, vor, welches die folgenden Schritte umfasst:
- Leiten eines zu filtrierenden, hefehaltigen Unfiltrats durch ein Tiefenfiltermedium aus lebensmittelzugelassenem Kunststoff, gekennzeichnet durch 11
- Leiten des Unfiltrats durch das Tiefenfiltermedium ohne Zugabe eines Filterhilfsmittels,
- Regenerieren und Reinigen des Tiefenfiltermediums durch
- Spülen des Tiefenfiltermediums mit Wasser, bis das Filtrat schaumfrei ist,
- alkalisches Spülen des Tiefenfiltermediums zur Regeneration desselben, und
- saures Spülen des Tiefenfiltermediums zur Neutralisation.

Unter dem Begriff "Tiefenfiltermedium" wird im Rahmen dieser Erfindung ein Filtermedium verstanden, dessen Porenweite oder Porengröße in Filtrationsrichtung abnimmt. Die Abnahme der Porenweite oder Porengröße in Filtrationsrichtung von einer Ausgangsporenweite oder -porengröße zu einer minimalen Porenweite oder -größe kann kontinuierlich oder stufenweise erfolgen. Tiefenfiltermedien an sich sind bekannt und beispielsweise von der Firma Pall unter der Bezeichnung Profile II und Nexis®, von der Firma Amazonfilters unter der Bezeichnung SupaSpun und SupaGard und von der Firma Eaton unter der Bezeichnung Protect PG kommerziell erhältlich. Zur Verwendung im erfindungsgemäßen Verfahren muss das Tiefenfiltermedium aus einem Kunststoff bestehen, der zur Verwendung mit Lebensmitteln zugelassen ist (siehe etwa die EG-Norm 1935). Geeignete Kunststoffe sind beispielsweise Polypropylen, Polyamid (Nylon®) und Polyester.

Gemäß einer bevorzugten Ausgestaltung des erfindungsgemäßen Verfahrens ist das Tiefenfiltermedium ein Mikrofaservlies mit in Filtrationsrichtung abnehmender Porenweite. Eine solche in Filtrationsrichtung abnehmende Porenweite lässt sich beispielsweise in dem Mikrofaservlies durch Fasern realisieren, deren Durchmesser in Filtrationsrichtung gesehen abnimmt. Mit anderen Worten, ein erster Bereich des Tiefenfiltermediums kann aus Fasern mit dickerem Durchmesser bestehen und in Filtrationsrichtung nachfolgende Bereiche des Tiefenfiltermediums können jeweils aus Fasern bestehen, deren Durchmesser geringer ist als der Durchmesser der Fasern, die den in Filtrationsrichtung vorgelagerten Bereich des Tiefenfiltermediums ausbilden. Anstelle eines aus Fasern bestehenden Tiefenfiltermediums sind aber auch andere poröse Körper denkbar, etwa in der Art der als Katalysatorträger verwendeten Wabenkörper. Solche Körper könnten beispielsweise durch Aufschäumen eines geeigneten Kunststoffs hergestellt werden.

Insbesondere dann, wenn das Tiefenfiltermedium aus Fasern in Gestalt eines Gewebes oder Vlieses ausgebildet ist, kann es vorzugsweise in Form einer Filterpatrone und somit als ein bis auf eine Zu- und Abfuhröffnung geschlossenes Bauteil bereitgestellt werden. Ein solches, beispielsweise mattenartig ausgestaltetes Tiefenfiltermedium ist in einer Filterpatrone vorzugsweise plissiert angeordnet, um auf geringem Raum eine hohe Filterfläche zur Verfügung zu stellen.

Das erfindungsgemäße Verfahren, welches die Hefe mittels eines regenerierbaren Tiefenfiltermediums ohne den Einsatz von Filterhilfsmitteln abscheidet, ermöglicht es unter Beibehaltung des von bekannten Filtersystemen gewohnten Filtrationsergebnisses die Investitionskosten um den Faktor 8 bis 10 gegenüber herkömmlichen Systemen abzusenken. Darüber hinaus sind die Unterhaltskosten eines nach dem erfindungsgemäßen Verfahren arbeitenden Filtersystems deutlich geringer, da das erfindungsgemäß eingesetzte Tiefenfiltermedium durch die Schritte des Spülens mit Wasser, des alkalischen Spülens und schließlich des sauren Spülens einfach und effektiv regeneriert und gereinigt werden kann, ohne dass beispielsweise Teile des Filtersystems demontiert und/oder turnusmäßig ersetzt werden müssen.

Bei bevorzugten Ausgestaltungen des erfindungsgemäßen Verfahrens wird der gewünschte Grad der Hefeabscheidung durch eine mehrstufige Filtration erreicht, d. h. der Schritt des Leitens des zu filtrierenden, hefehaltigen Unfiltrats durch das Tiefenfiltermedium umfasst mehrere Stufen, wobei jede auf eine Stufe folgende Stufe eine minimale Porenweite hat, die kleiner ist als die minimale Porenweite der vorhergehenden Stufe. Beispielsweise kann das Tiefenfiltermedium drei Stufen umfassen, wobei eine erste Stufe eine minimale Porenweite von etwa 5 µm, vorzugsweise von etwa 3 µm haben kann, eine zweite Stufe eine minimale Porenweite von etwa 1 µm haben kann und eine dritte Stufe eine minimale Porenweite von etwa 0,5 µm haben kann. In jeder Stufe einer solchen mehrstufigen Hefeabscheidung wird somit ein Tiefenfiltermedium verwendet, dessen Porenweite oder Porengröße in Filtrationsrichtung abnimmt. Die Porenweiten- oder Porengrößenbereiche jeder einzelnen Stufe können mit der einer vorhergehenden oder nachfolgenden Stufe überlappen, jedoch weist jede auf eine Stufe folgende Stufe eine minimale Porenweite auf, die kleiner ist als die minimale Porenweite der vorhergehenden Stufe. Wie viele Stufen zur Hefeabscheidung vorgesehen werden hängt in der Regel von den im Einzelfall bestehenden Anforderungen ab, beispielsweise davon, wie klar das schlussendlich in Flaschen abgefüllte Getränk sein soll.

Weil ein zu hoher Eiweißgehalt beispielsweise im Bier bei längerer Lagerung zu einer unerwünschten Trübung des Biers führt, ist es bekannt, zur Reduktion des Eiweißgehalts dem Sud Stabilisierungsmittel zuzusetzen, beispielsweise Silikagele oder Xerogele, die zur Eiweißabsorption dienen. Solche Stabilisierungsmittel müssen jedenfalls dann, wenn sie nicht in nur geringen Mengen zugesetzt worden sind, aus dem Unfiltrat abgeschieden werden, bevor das Unfiltrat durch das Tiefenfiltermedium geleitet wird. Gemäß einer Variante des erfindungsgemäßen Verfahrens kann deshalb dem Schritt des Leitens des zu filtrierenden, hefehaltigen Unfiltrats durch das Tiefenfiltermedium ein Abscheiden eines Stabilisierungsmittels vorgeschaltet sein. Das Abscheiden von Stabilisierungsmittel kann durch Verwendung von Beutelfiltern, Rückspülfiltern oder Partikelfiltern erreicht werden, alternativ ist die Abscheidung von Stabilisierungsmitteln auch mittels einer Zentrifuge möglich.

Je nach den Ansprüchen an die Lagerfähigkeit eines Getränks, z. B. eines Bieres kann es wünschenswert sein, dass auf den Schritt des Leitens des zu filtrierenden, hefehaltigen Unfiltrats durch das Tiefenfiltermedium eine Entkeimungsfiltration folgt. Wenn der Schritt des Leitens des zu filtrierenden, hefehaltigen Unfiltrats durch das Tiefenfiltermedium mehrstufig ausgeführt ist, dann können in besonders vorteilhafter Weise die auf die erste Stufe folgende(n) Stufe(n) nicht nur zur Hefeabscheidung, sondern gleichzeitig auch zur Entkeimungsfiltration dienen, wenn die minimale Porenweite entsprechend niedrig gewählt wird, z. B. ≤ 1 µm oder ≤ 0,5 µm.

Bei bevorzugten Ausgestaltungen des erfindungsgemäßen Verfahrens wird der Schritt des Spülens des Tiefenfiltermediums mit Wasser so ausgeführt, dass die Temperatur des zugeführten Wassers über die Zeitdauer des Spülens ansteigt. Beispielsweise kann der Spülvorgang zunächst mit kaltem Wasser begonnen werden und die Temperatur des zum Spülen verwendeten Wassers dann nach und nach erhöht werden. In jedem Fall zeigt ein schaumfreies Filtrat, dass der Spülvorgang beendet werden kann, weil das zur Schaumbildung führende Eiweiß aus dem Tiefenfiltermedium ausgewaschen worden ist.

Zum alkalischen Spülen des Tiefenfiltermediums kann beispielsweise eine 2-prozentige Lauge, z. B. eine Natronlauge, verwendet werden. Das alkalische Spülen löst die im Tiefenfiltermedium vorhandenen Hefebestandteile auf, ohne das Tiefenfiltermedium selbst zu beschädigen. Es ist erfindungsgemäß nicht notwendig, das alkalische Spülen in Form eines Rückspülens des Tiefenfiltermediums durchzuführen, vielmehr kann das alkalische Spülen ohne weiteres in Filtrationsrichtung erfolgen, was den Anlagenaufbau vereinfacht und verbilligt.

Nach dem alkalischen Spülen des Tiefenfiltermediums findet erfindungsgemäß ein saures Spülen des Tiefenfiltermediums statt, beispielsweise mit einer 0,5 bis 1-prozentigen Säure, z. B. Phosphorsäure oder Salpetersäure. Zur Einsparung von saurem Spülmedium und zur Vermeidung etwaiger unerwünschter Nebenreaktionen kann vor dem sauren Spülen des Tiefenfiltermediums ein erneutes Spülen desselben mit Wasser vorgeschaltet sein. Nach dem zur Neutralisation dienenden sauren Spülen des Tiefenfiltermediums steht das Filtersystem zur erneuten Verwendung bereit, ohne dass das Filtersystem geöffnet werden musste und ohne dass Filterelemente oder ähnliches ausgetauscht werden mussten.

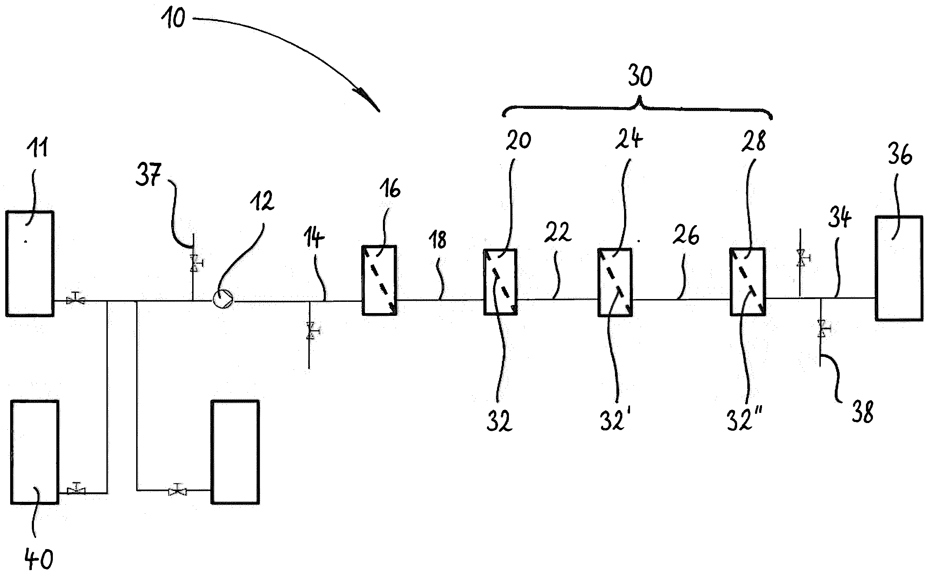

Ein Ausführungsbeispiel eines erfindungsgemäßen Verfahrens wird im Folgenden anhand der schematischen, einzigen Figur näher erläutert, die ein Filtersystem zeigt, das nach dem erfindungsgemäßen Verfahren funktioniert.

In der Figur ist ein allgemein mit 10 bezeichnetes Filtersystem mit einem Unfiltrattank 11 gezeigt, in dem sich zu filtrierende, hefehaltige Flüssigkeit befindet, z. B. Bier. Zur Abtrennung gegebenenfalls zugegebener Stabilisierungsmittel wird das Unfiltrat mittels einer Pumpe 12 durch eine Leitung 14 zunächst durch einen Vorfilter 16 geführt, der hier als Beutelfilter ausgestaltet ist. Wenn das Unfiltrat kein oder nur sehr wenig Stabilisierungsmittel enthält, kann auf den Vorfilter 16 verzichtet werden. Aus dem Vorfilter 16 gelangt das nun stabilisierungsmittelfreie Unfiltrat durch eine Leitung 18 in eine erste Filterstufe 20 eines allgemein mit 30 bezeichneten Hauptfilters, in der das Unfiltrat durch ein Tiefenfiltermedium 32 mit einer minimalen Porenweite von 3 µm geleitet wird.

Nach Durchlaufen der ersten Filterstufe 20 gelangt die zu filtrierende Flüssigkeit durch eine Leitung 22 in eine zweite Filterstufe 24, in der die zu filtrierende Flüssigkeit ein Tiefenfiltermedium 32' durchläuft, das eine minimale Porenweite von 1 µm hat.

Aus der zweiten Filterstufe 24 fließt die zu filtrierende Flüssigkeit durch eine Leitung 26 in eine dritte Filterstufe 28, in der sie ein Tiefenfiltermedium 32" passiert, welches eine minimale Porenweite von 0,5 µm hat. Aus der dritten Filterstufe 28 des Hauptfilters 30 strömt das Filtrat durch eine Leitung 34 in einen Filtrattank 36, aus dem es z. B. zu einer Abfüllanlage (nicht gezeigt) geleitet werden kann.

Zur Reinigung und Regeneration des Hauptfilters 30 und insbesondere der Tiefenfiltermedien 32, 32' und 32" wird zunächst Wasser aus einem Spülanschluss 37 durch die Filterstufen 20, 24 und 28 gespült, bis das aus der letzten Filterstufe 28 an einem Ausgang 38 austretende Filtrat schaumfrei ist, was bedeutet, dass das im Filtersystem und insbesondere in den Tiefenfiltermedien 32, 32' und 32" enthaltene Eiweiß vollständig oder zumindest weitestgehend ausgewaschen worden ist.

Sodann wird aus einem Laugentank 40 eine Lauge, z. B. eine 2-prozentige Natronlauge, durch das Filtersystem und insbesondere durch die Filterstufen 20, 24 und 28 und damit durch die Tiefenfiltermedien 32, 32' und 32" geleitet, um die in den Tiefenfiltermedien vorhandenen Heferückstände aufzulösen und dadurch die Tiefenfiltermedien zu regenerieren.

Nach Abschluss der durch das alkalische Spülen erfolgten Regeneration der Tiefenfiltermedien 32, 32' und 32" wird das Filtersystem erneut mit Wasser gespült, welches über den Spülanschluss 37 zugeführt wird. Zur Neutralisation etwaiger Laugenrückstände im Filtersystem wird sodann aus einem Neutralisationstank 42 eine 0,5 bis 1-prozentige Säure, etwa Phosphorsäure, Salpetersäure oder auch Mischungen daraus, durch das Filtersystem und insbesondere die Filterstufen 20, 24 und 28 mit ihren Tiefenfiltermedien 32, 32' und 32" geleitet und durch den Ausgang 38 abgelassen. Danach steht das Filtersystem zur erneuten Hefeabscheidung aus hefehaltigen Flüssigkeiten bereit.

## Patentansprüche

1. Verfahren zur Hefeabscheidung aus hefehaltigen Flüssigkeiten, insbesondere Getränken, mit den Schritten:
- Leiten eines zu filtrierenden, hefehaltigen Unfiltrats durch ein Tiefenfiltermedium aus lebensmittelzugelassenem Kunststoff,
**gekennzeichnet durch**
- Leiten des Unfiltrats durch das Tiefenfiltermedium ohne Zugabe eines Filterhilfsmittels,
- Regenerieren und Reinigen des Tiefenfiltermediums durch
-- Spülen des Tiefenfiltermediums mit Wasser, bis das Filtrat schaumfrei ist,
- alkalisches Spülen des Tiefenfiltermediums zur Regeneration desselben, und
-- saures Spülen des Tiefenfiltermediums zur Neutralisation.

2. Verfahren nach Anspruch 1,
**dadurch gekennzeichnet, dass** das Tiefenfiltermedium ein Mikrofaservlies mit in Filtrationsrichtung abnehmender Porenweite ist.

3. Verfahren nach Anspruch 1 oder 2,
**dadurch gekennzeichnet, dass** der Schritt des Leitens des zu filtrierenden, hefehaltigen Unfiltrats durch das Tiefenfiltermedium mehrere Stufen umfasst, wobei jede auf eine Stufe folgende Stufe eine minimale Porenweite hat, die kleiner ist als die minimale Porenweite der vorhergehenden Stufe.

4. Verfahren nach Anspruch 3,
**dadurch gekennzeichnet, dass** der Schritt des Leitens des zu filtrierenden, hefehaltigen Unfiltrats durch das Tiefenfiltermedium drei Stufen umfasst, wobei eine erste Stufe eine minimale Porenweite von etwa 5 µm, vorzugsweise von etwa 3 µm hat, eine zweite Stufe eine minimale Porenweite von etwa 1 µm hat, und eine dritte Stufe eine minimale Porenweite von etwa 0,5 µm hat.

5. Verfahren nach einem der vorhergehenden Ansprüche,
**dadurch gekennzeichnet, dass** dem Schritt des Leitens des zu filtrierenden, hefehaltigen Unfiltrats durch das Tiefenfiltermedium ein Abscheiden eines Stabilisierungsmittels vorgeschaltet ist.

6. Verfahren nach einem der vorhergehenden Ansprüche,
**dadurch gekennzeichnet, dass** auf den Schritt des Leitens des zu filtrierenden, hefehaltigen Unfiltrats durch das Tiefenfiltermedium eine Entkeimungsfiltration folgt.

7. Verfahren nach Anspruch 6 in Verbindung mit Anspruch 3 oder 4,
**dadurch gekennzeichnet, dass** eine zweite oder dritte Stufe des Schritts des Leitens des zu filtrierenden, hefehaltigen Unfiltrats durch das Tiefenfiltermedium zugleich als Entkeimungsfiltration fungiert.

8. Verfahren nach einem der vorhergehenden Ansprüche,
**dadurch gekennzeichnet, dass** der Schritt des Spülens des Tiefenfiltermediums mit Wasser mit über die Zeitdauer des Spülens steigender Temperatur durchgeführt wird.

9. Verfahren nach einem der vorhergehenden Ansprüche,
**dadurch gekennzeichnet, dass** dem Schritt des sauren Spülens des Tiefenfiltermediums zum Neutralisieren ein erneutes Spülen des Tiefenfiltermediums mit Wasser vorgeschaltet ist.

## Claims

1. Method for separating yeast from yeast-containing liquids, in particular beverages, comprising the steps:
- conducting a yeast-containing unfiltrate to be filtered through a depth filter medium of food-grade plastics material,
**characterised by**
- conducting the unfiltrate through the depth filter medium without the addition of a filter aid,
- regenerating and cleaning the depth filter medium by
-- flushing the depth filter medium with water until the filtrate is foam-free,
-- alkali flushing the depth filter medium for regeneration thereof, and
-- acid flushing the depth filter medium for neutralisation.

2. Method according to claim 1,
**characterised in that** the depth filter medium is a microfibre nonwoven with a decreasing pore size in the filtration direction.

3. Method according to claim 1 or 2,
**characterised in that** the step of conducting the yeast-containing unfiltrate to be filtered through the depth filter medium comprises multiple stages, wherein each stage following a stage has a minimum pore size which is smaller than the minimum pore size of the preceding stage.

4. Method according to claim 3,
**characterised in that** the step of conducting the yeast-containing unfiltrate to be filtered through the depth filter medium comprises three stages, wherein a first stage has a minimum pore size of approximately 5 µm, preferably of approximately 3 µm, a second stage has a minimum pore size of approximately 1 µm, and a third stage has a minimum pore size of approximately 0.5 µm.

5. Method according to any one of the preceding claims,
**characterised in that** the step of conducting the yeast-containing unfiltrate to be filtered through the depth filter medium is preceded by the separation of a stabilising agent.

6. Method according to any one of the preceding claims,
**characterised in that** the step of conducting the yeast-containing unfiltrate to be filtered through the depth filter medium is followed by sterile filtration.

7. Method according to claim 6 in conjunction with claim 3 or 4,
**characterised in that** a second or third stage of the step of conducting the yeast-containing unfiltrate to be filtered through the depth filter medium at the same time serves as sterile filtration.

8. Method according to any one of the preceding claims,
**characterised in that** the step of flushing the depth filter medium with water is carried out at a temperature that increases over the duration of the flushing.

9. Method according to any one of the preceding claims,
**characterised in that** the step of acid flushing the depth filter medium for neutralisation is preceded by flushing the depth filter medium with water again.

## Revendications

1. Procédé de séparation de la levure à partir de liquides contenant de la levure, en particulier des boissons, comprenant les étapes consistant à :
- guider une substance à filtrer contenant de la levure à travers un médium filtrant en profondeur en matière plastique de qualité alimentaire,
**caractérisé par**
- le guidage de la substance à filtrer à travers le médium filtrant en profondeur sans ajout d'un médium filtrant auxiliaire,
- régénérer et nettoyer le médium filtrant en profondeur par
-- rinçage du médium filtrant en profondeur avec de l'eau, jusqu'à ce que la substance filtrée soit exempte de mousse,
-- rinçage alcalin du médium filtrant en profondeur pour sa régénération, et
-- rinçage acide du médium filtrant en profondeur pour la neutralisation.

2. Procédé selon la revendication 1, **caractérisé en ce que** le médium filtrant en profondeur étant un non-tissé en microfibre dont la largeur des pores décroît dans le sens de filtration.

3. Procédé selon la revendication 1 ou la revendication 2, **caractérisé en ce que** l'étape consistant à guider la substance à filtrer contenant de la levure à travers le médium filtrant en profondeur comprend plusieurs étapes, chaque étape suivant l'étape suivante présente une largeur de pore minimale qui est inférieure à la largeur de pore minimale de l'étape précédente.

4. Procédé selon la revendication 3, **caractérisé en ce que** l'étape du guidage de la substance à filtrer contenant de la levure à travers le médium filtrant en profondeur comprend trois étapes, une première étape ayant une largeur de pores minimale d'environ 5 µm, de préférence d'environ 3 µm, une deuxième étape ayant une largeur de pores minimale d'environ 1 µm, et une troisième étape ayant une largeur minimale de pores d'environ 0,5 µm.

5. Procédé selon l'une quelconque des revendications précédentes, **caractérisé en ce que** l'étape du guide de la substance à filtrer contenant de la levure à travers le médium filtrant en profondeur est précédée d'une séparation d'un moyen de stabilisation.

6. Procédé selon l'une quelconque des revendications précédentes, **caractérisé en ce qu'**une filtration de désinfection fait suite à l'étape du guidage de la substance à filtrer contenant de la levure.

7. Procédé selon la revendication 6 en association avec la revendication 3 ou la revendication 4, **caractérisé en ce qu'**une deuxième ou une troisième étape du guidage de la substance à filtrer contenant de la levure à travers le médium filtrant en profondeur fait office en même temps de filtration de désinfection.

8. Procédé selon l'une quelconque des revendications précédentes, **caractérisé en ce que** l'étape de rinçage du médium filtrant en profondeur avec de l'eau est effectuée à une température croissante sur la durée du rinçage.

9. Procédé selon l'une quelconque des revendications précédentes, **caractérisé en ce que** l'étape du rinçage acide du médium filtrant en profondeur pour neutraliser est précédée d'un autre rinçage du médium filtrant en profondeur avec de l'eau.
